# EUROPEAN PATENT APPLICATION

(11) **EP 2 193 842 A1**
(43) Date of publication of application: **09.06.2010**
(21) Application number: 08021306.9
(22) Date of filing: 08.12.2008
(51) Int. Cl.: B01J 23/78, B01J 35/10, C07C 1/04, C10G 2/00, B01J 23/745, B01J 37/03, B01J 27/02, B01J 27/185, B01J 27/043, B01J 23/58

(54) **Preparation of a hydrocarbon synthesis catalyst**

(71) Applicant: Sasol Technology (Pty) Ltd, 2000 Johannesburg (ZA)
(72) Inventor: Crous, Reinier, SW5 Ext. 2, 1911 (ZA); Bromfield, Tracey Carolyn, Vanderbijlpark 1911 (ZA)
(74) Representative: Kador & Partner

(57) **Abstract**

The present invention therefore provides a process for the preparation of a hydrocarbon synthesis catalyst, especially a Fischer-Tropsch catalyst, comprising the following steps:
(a) providing a solution of iron or a suspension of a precipitated, non-calcined iron-containing solid in a solvent in which a precursor of a promoter P1 is present, the promoter P1 comprising one or more element(s) selected from the group of boron, germanium, nitrogen, phosphorus, arsenic, antimony, sulphur, selenium and tellurium,
(b) removing the solvent from the solution or suspension, and
(c) subjecting the product of step (b) to a calcination treatment,

wherein
- the amount of the precursor of promoter P1 is selected so that the one or more element(s) selected from boron, germanium, nitrogen, phosphorus, arsenic, antimony, sulphur, selenium and tellurium is(are) present in an amount of at least 0.02 g/100 g Fe in the catalyst, and
- a precursor of an alkali metal- and/or alkaline earth metal containing promoter P2 is added before, after or during any of the steps of the process so that promoter P2 is present in the catalyst,

to a catalyst obtainable by such a process, to a hydrocarbon synthesis process carried out in the presence of such a catalyst and to the use of the catalyst in a hydrocarbon synthesis process.

## Description

This invention relates to the preparation of a hydrocarbon synthesis catalyst comprising Fe as catalytically active metal, to the catalyst obtained by said process, to a hydrocarbon synthesis process in the presence of the catalyst and to the use of the catalyst in a hydrocarbon synthesis process.

Hydrocarbon synthesis by the Fischer-Tropsch process is known to produce a variety of products including paraffins, olefins, and oxygenates with different carbon chain lengths and isomers. One of the most serious drawbacks of the Fischer-Tropsch synthesis is that the product spectrum usually obtained is broad as predicted by the Schulz-Flory product distribution model.

The chemical industry uses as feedstock mainly short chain olefins, particularly the C₂-C₄ olefins. These olefins are normally derived from various crude oil sources. A synthetic route based on the Fischer-Tropsch technology would be highly desirable from an economic point of view as the reliance on crude oil would be eliminated.

It is known in the art that to some extent selectivity is a function of conversion, and that selectivity to lower molecular weight hydrocarbons can be enhanced by operating at process conditions for low conversion of CO. Preferred methods for lowering the conversion include increasing the space velocity and lowering the temperature. However, low conversions of valuable feed gas are undesirable for commercial applications because of low yields for the desired products.

It is also known in the art that methane production can be suppressed by dramatically lowering the H₂:CO ratio of the feed gas. This has the detrimental effect of increasing the selectivity for higher molecular weight hydrocarbons.

A further approach to enhance the yield of C₂-C₄ olefins in a Fischer-Tropsch synthesis without simultaneously producing large quantities of the corresponding C₂-C₄ paraffins was the development of new catalysts which selectively produce the short chain olefins and simultaneously minimize the production of short chain paraffins, especially methane.

Many catalyst examples in the art relate to improvements in the selectivity to a desirable product range of C₂-C₄ compounds. For example, in US 4,199,522 and US 4,151,190 a method is disclosed for increasing the selectivity to C₂-C₄ olefins by using a catalyst comprising at least one member of the group of metals, oxides or sulphides of molybdenum, tungsten, rhenium, ruthenium, nickel, palladium, rhodium, osmium, iridium, and platinum, and at least one member of the group of hydroxides, oxides, or salts of alkaline or alkaline earth metals.

In US 4,539,334 the addition of phosphorus (in the form of a volatile phosphorous precursor) to a conventional Fischer-Tropsch catalyst is claimed to improve the selectivity of said catalyst to C₂-C₄ olefins. However, these results were obtained at relatively low conversions of CO at a H₂:CO ratio of ≤ 1.

In a study by van der Baan et al. (See Applied Catalysis, [1982]. 2, pp. 273-288) a Fe/Mn catalyst containing sulphur was prepared by impregnating the calcined Fe/Mn oxide with ammonium sulphate solution. Below 350 °C the main product of the catalyst is methane. At 350 °C the ethylene selectivity is higher than the methane selectivity. However, the methane formation can still be reduced.

In a study by Wu et al. (See Fuel, [2004], 83, pp. 205-212) a precipitated iron Fischer-Tropsch catalyst containing sulphur was prepared by using ferrous sulphate as precursor. It was shown that small amounts of sulphur may promote the catalyst by increasing activity and improving the heavier hydrocarbon selectivity.

More recently, Zhang et al. (see J. Nat. Gas Chem., [2007], 16, 377-381) studied the influence of several anions on the performance of Fe-based Fischer-Tropsch catalysts. The catalyst used in this investigation was of the composition Fe:Mn:K:activated carbon = 25:20:5:50 by weight. From this study it was revealed that the addition of 500 ppm of anions lowered the catalytic activity of the catalyst for Fischer-Tropsch synthesis as well as the yield of light olefins. The selectivity for methane was also lowered by the presence of the anions.

WO 01/97968 discloses the preparation of an iron-based precipitated FT catalyst. The calcined catalyst of Example 2 includes "impurity levels" of 0.03 g SO₄/100 g Fe, which translates to 0.01 g S/100 g Fe. It is shown that the catalyst had only a low selectivity to C₂-C₄ olefins with high methane selectivity.

Finally, in US 6,653,357 a method for conducting a high temperature Fischer-Tropsch synthesis is disclosed with which the selectivity profile of the lower olefins is improved by injecting a promoter-carrying compound directly into the reactor medium thereby counteracting the poisonous effect of sulphur.

However, in spite of the various approaches to obtain an improved yield of short chain products described above there is still the need for improved Fischer-Tropsch catalysts tailored to produce a high amount of C₂-C₄ products, especially C₂-C₄ olefins, while keeping the methane formation at a low level.

It is thus the object of the present invention to provide a process for the preparation of a hydrocarbon synthesis catalyst which has an improved selectivity for C₂-C₄ products, especially C₂-C₄ olefins, with simultaneous suppression of excess methane formation. Simultaneously, the catalyst should have high activity and should allow for high CO conversion rates.

It has surprisingly been found that such a catalyst can be provided by a process in which a catalyst is produced which contains iron as a catalytically active metal, an alkali metal and/or alkaline-earth metal containing promoter and, in addition, a further promoter which is selected from the group of non-metals and semi-metals (Metalloids) found in groups 13 to 16 of the Periodic Table of the Elements and which is incorporated into the catalyst before calcination.

The present invention therefore provides a process for the preparation of a hydrocarbon synthesis catalyst, especially a Fischer-Tropsch catalyst, comprising the following steps:
(a) providing a solution of iron or a suspension of a precipitated, non-calcined iron-containing solid in a solvent in which a precursor of a promoter P1 is present, the promoter P1 comprising, or consisting of, one or more element(s) selected from the group of boron, germanium, nitrogen, phosphorus, arsenic, antimony, sulphur, selenium and tellurium,
(b) removing the solvent from the solution or suspension, and
(c) subjecting the product of step (b) to a calcination treatment,
wherein
- the amount of the precursor of promoter P1 is selected so that the one or more element(s) selected from boron, germanium, nitrogen, phosphorus, arsenic, antimony, sulphur, selenium and tellurium is(are) present in an amount of at least 0.02 g/100 g Fe in the catalyst, and
- a precursor of an alkali metal- and/or alkaline earth metal containing promoter P2 is added before, after or during any of the steps of the process so that promoter P2 is present in the catalyst.

Unless otherwise mentioned the term "iron" as used herein is intended to denote iron in any of its oxidation states, i.e. including Fe²⁺ and Fe³⁺. A solution of iron as mentioned herein will usually comprise iron in one of its ionic forms.

The term "precursor of promoter" includes the exceptional case in which the precursor and the promoter derived therefrom and present in the catalyst in its reduced form have the same chemical composition.

The catalyst obtainable by the process according to the invention allows for a dramatic increase in selectivity to light hydrocarbons with a simultaneous suppression of the expected increase in methane formation when used in hydrocarbon synthesis, especially Fischer-Tropsch synthesis, in which a feed gas composed of H₂ and at least one carbon oxide is converted at elevated temperature and pressure. Furthermore, the light fraction of the obtained hydrocarbons consists mostly of valuable olefins and alcohols with minimal paraffin formation. Still further, the catalysts show a good activity and synthesis gas conversion.

The solvent is removed in step (b) to such an extent that a dry or wet solid, or a slurry is obtained. In any case, in the solid thus formed the precursor of promoter P1 and iron are homogenously distributed and, hence, also in the final catalyst structure promoter P1 is homogeneously distributed, i.e. not only attached to the surface.

Preferably, the solid or slurry obtained in step (b) is, after optional further drying, directly subjected to step (c) without that any further intermediate steps are performed.

However, the solid or slurry obtained in step (b) may also be resuspended in a solvent or impregnated with a liquid before it is, after optional further drying, subjected to step (c).

Preferably, the solvent present in the solution or suspension of step (a) is an aqueous solvent.

The solution of iron in a solvent in step (a) may be prepared in any known manner.

The solution of iron in a solvent may be formed by dissolving an iron salt in the solvent, preferably aqueous solvent, said iron salt is preferably selected from iron nitrate, iron acetate, iron oxalate, iron sulphate, iron chloride, their hydrates and mixtures thereof, more preferably the iron salt is iron nitrate or a hydrate thereof, and most preferably the iron salt is iron nitrate nonahydrate (Fe(NO₃)₃ · 9 H₂O).

The solution comprising iron may also be formed by dissolving a compound comprising at least 90 wt.-% elemental iron, more preferably at least 95 wt.-% elemental iron, even more preferably at least 98 wt.-% elemental iron and most preferably 99.5 wt.-% elemental iron in an aqueous acid.

Preferably, said aqueous acid is selected from aqueous inorganic acids, more preferably selected from aqueous solutions of nitric acid, sulphuric acid, hydrochloric acid or mixtures thereof and most preferably, the acid is nitric acid.

Preferably, said aqueous inorganic acid has a concentration of at least 15 wt.% in water, more preferably of at least 50 wt.% in water and most preferably of at least 60 wt.% in water.

Said aqueous inorganic acid preferably has a concentration of not more than 90 wt.% in water, more preferably not more than 80 wt.% in water.

In a preferred embodiment of the process of the invention, in step (a) a solution of iron in a solvent is provided in which a precursor of a promoter P1 is present, and then a suspension is obtained by forming a precipitate containing iron and the precursor of promoter P1 from the solution before step (b) is carried out.

The precipitation from an iron-solution containing a precursor of promoter P1 is a co-precipitation wherein besides iron at least a part of the precursor of promoter P1 is present in the precipitate.

The precipitation may be effected by reduction of the temperature or by using a precipitant.

Preferably, the precipitation is effected using a precipitant, i.e. a chemical agent which initiates precipitation.

The precipitation may be effected by addition of the solution containing iron and the precursor of promoter P1 to the precipitant or, preferably, by addition of the precipitant to the solution containing iron the precursor of promoter P1.

In the embodiment wherein in step (a) an aqueous solvent is used, the precipitation is preferably effected by the addition of an alkaline compound or a solution thereof to the solution containing iron and the precursor of promoter P1. More preferably, the precipitant is selected from solutions, preferably aqueous solutions, of alkaline compounds, more preferably is selected from solutions of hydroxides, even more preferably selected from solutions of alkali metal hydroxides, ammonium hydroxide or mixtures thereof and most preferably is a solution of ammonium hydroxide.

Preferably, the precipitant is used in an amount so that a pH value of the solvent of at least 5.0, more preferably at least 6.0 and most preferably of at least 6.5 is attained.

Furthermore, preferably the pH value obtained by the addition of the precipitant does not exceed 9.0, more preferably does not exceed 8.0 and most preferably does not exceed 7.5.

The precipitation is preferably carried out at a temperature of at least 0 °C, more preferably of at least 10 °C and most preferably of at least 20 °C.

Preferably, the precipitation is carried out at a temperature of not more than 100 °C, more preferably of not more than 70 °C and most preferably of not more than 40 °C.

Preferably, at least 80 mol%, more preferably at least 90 mol% and most preferably at least 95 mol% of the iron in the precipitate is present in the form of ferrihydrite.

In case a suspension of a precipitated, non-calcined iron-containing solid in a solvent is used in step (a), this suspension may be prepared in any known manner, for example by precipitating the solid from an iron solution by the use of a precipitating agent and the so-prepared suspension is used in step (a). In particular, the suspension can be prepared in according to any embodiments of the precipitation process described above.

However, it is also possible that the suspension of a precipitated, non-calcined iron-containing solid in a solvent is prepared by providing a solution of iron in a solvent followed by forming a precipitate containing iron from the solution, removing the solvent to obtain a dry or wet solid, or a slurry, and then re-suspending the solid in a solvent.

In any case, the precipitated iron-containing solid must be non-calcined, i.e. must not have been subjected to a calcination treatment where heat is applied to the solid in an oxygen atmosphere so that iron oxides are formed in the solid.

Usually, a calcination treatment is performed at a temperature of at least 200 °C for a time of at least ½ hour.

The removal of the solvent in step (b) may be effected by spray-drying or, preferably, by evaporating the solvent by the application of heat, i.e. by heating.

In a typical spray-drying operation, the solution or suspension is pumped into the chamber of the spray-drying apparatus through an atomizer or series of atomizers. The simplest type is a single fluid atomizer in which the solution or suspension to be dried is pumped at high pressures through small nozzles. A more complex atomizer is a spinning disk system in which the solution or suspension to be dried is carefully fed onto a disk that is spinning at 10,000-30,000 rpm. The high speed rotation causes the liquid to thin and form droplets at the edge of the disk. A third commonly used method for atomization is a two-fluid nozzle. In this case, there are two closely spaced nozzles. One nozzle is used to deliver the solution or suspension and the second nozzle directs gas at high pressure onto the liquid stream causing it to atomize. In all cases, the atomized liquid is then dried by the hot gas flowing through the drying chamber.

For rapid drying, a high inlet temperature is desired, although thermal stability of the product or limitations on the air heaters supplying the hot air limits the upper end of inlet temperatures. For most drying, the outlet temperature provides a measure of the final product temperature and an indication of the utilization of the energy in the inlet air for drying. Clearly, for the most economical drying, a low outlet temperature would be desired. However, in many instances, a low outlet temperature leaves the product with a moisture content that is too high.

Preferably, the outlet temperature is not more than 180 °C, more preferably not more than 160 °C and even more preferably not more than 140 °C.

The outlet temperature is preferably at least 80 °C, more preferably at least 95 °C, even more preferably at least 110 °C.

The inlet temperature is preferably not more than 500 °C, more preferably not more than 450 °C and most preferably not more than 400 °C.

Preferably, the inlet temperature is at least 250 °C, more preferably at least 300 °C.

Preferably, spray-drying is performed at a pressure of at least 5.0 MPa, more preferably of at least 10 MPa and most preferably of at least 15

MPa.

Spray-drying is preferably performed at a pressure of not more than 40.0 MPa, more preferably of not more than 30 MPa and most preferably of not more than 25 MPa before subjected to spray-drying.

A carrier gas may be used in spray-drying. Preferably, said carrier gas is selected from air, He, Ne, Ar, Kr, Xe, N₂, or mixtures thereof and most preferably from air or nitrogen.

If spray-drying is used in step (b), it is preferred that a solution comprising iron and the precursors of promoters P1 and P2 is prepared in step (a) and is then spray-dried in step (b).

In the preferred option of the removal of the solvent in step (b) by heating, the temperature is preferably at least 110 °C, more preferably at least 120 °C and most preferably at least 130 °C.

Furthermore, the heating temperature preferably is not higher than 200 °C, more preferably is not higher than 150 °C and most preferably is not higher than 140 °C.

Heating is preferably carried out for at least 9 hours, more preferably for at least 12 hours and most preferably for at least 14 hours.

Preferably, heating is carried out for not more than 25 hours, more preferably for not more than 20 hours, and most preferably for not more than 18 hours.

Preferably, in the process of the invention, the calcination treatment in step (c) is performed at a temperature of 200 °C or more, more preferably at a temperature of 250 °C or more and most preferably at a temperature of 300 °C or more.

The temperature in the calcination treatment in step (c) preferably is not higher than 600 °C, more preferably is not higher than 500 °C and most preferably is not higher than 450 °C.

The calcination treatment in step (c) is preferably carried out for at least 30 min, more preferably for at least 1.0 hour, even more preferably for at least 2.0 hours and most preferably for at least 3.0 hours.

Preferably, the calcination treatment in step (c) is performed for at most 8.0 hours, preferably at most 6.0 hours, and most preferably at most 5.0 hours.

Preferably, the surface area of the product subjected to the calcination treatment in step (c) is at least 50 m²/g and more preferably is at least 80 m²/g before step (c) is carried out. Furthermore, preferably, the surface area of the product subjected to the calcination treatment in step (c) is not higher than 500 m²/g.

In the calcination treatment in step (c), usually the surface area of the treated product is lowered. Furthermore, as a calcination treatment is carried out in the presence of an oxygen atmosphere, e.g. in air, iron oxides are formed in the treated product.

After the calcination treatment in step (c), the catalyst is obtained (in unreduced form).

The surface area of the catalyst in its unreduced form is preferably 80 m²/g or less, more preferably is 50 m²/g or less, still more preferably is 40 m²/g or less, and most preferably is 35 m²/g or less.

Furthermore, the catalyst in its unreduced form preferably has a surface area of 10 m²/g or more, more preferably of 25 m²/g or more.

Preferably, after calcination step (c) at least 80 mol%, more preferably at least 90 mol% and most preferably at least 95 mol% of the iron contained in the catalyst in its unreduced form is in the form of hematite.

Preferably, the process further comprises the following step (d) after step (c):
(d) the product of step (c) is brought into a particulate form in which more than 90 wt.% of the particles have a particle size in the range of 10 to 250 µm, preferably have a particle size in the range of 20 to 180 µm and most preferably have a particle size in the range of 38 to 150 µm.

Step (d) is preferably carried out by crushing the product of step (c) and sieving it to the desired particle distribution.

In the process of the invention, the precursor of an alkali metal- and/or alkaline earth metal containing promoter P2 may be added to the catalyst precursor at any stage of the process.

For example, the precursor of promoter P2 may be
- present in the solution or the suspension of step (a) before step (b) is carried out; and/or
- added to the product of step (b) before step (c) is carried out; and/or
- added to the catalyst in its unreduced form after step (c) or (d)

Preferably, the precursor of promoter P2 is added before step (c) is carried out, more preferably, the precursor of promoter P2 is added before step (b) is carried out.

In the preferred embodiment of the process of the invention wherein in step (a) a solution of iron in a solvent is provided in which a precursor of a promoter P1 is present, and then a suspension is obtained by forming a precipitate containing iron and the precursor of promoter P1 from the solution before step (b) is carried out, preferably, the precursor of promoter P2 is added to the suspension containing the precipitate before step (b) is carried out, i.e. preferably the process comprises the following steps:
(a) providing a solution comprising iron and a precursor of a promoter P1, the promoter P1 comprising, or consisting of, one or more element(s) selected from the group of boron, germanium, nitrogen, phosphorus, arsenic, antimony, sulphur, selenium and tellurium, in a solvent, obtaining a suspension by forming a precipitate containing iron and the precursor of promoter P1 from the solution, and adding a precursor of an alkali metal- and/or alkaline-earth metal containing promoter P2 to the suspension;
(b) removing the solvent from the suspension; and
(c) subjecting the product of step (b) to a calcination treatment.

In addition to the promoters described above, the catalyst may also contain other promoters. Thus, the precursors of certain promoters, for example Al, Ti and Cr may be added during the preparation process.

However, preferably, no further promoters besides P1 and P2 or their precursors are added after the calcination treatment in step (c). More preferably, no promoters besides P1 and P2 or their precursors at all are added during the preparation process.

Preferably, in the catalyst obtainable by the process of the invention Fe is the only metal which is catalytically active for hydrocarbon synthesis. Thus, preferably, no additional catalytically active metal or one of its precursors is added during the preparation process.

Preferably, promoter P1 comprises, or consists of, one or more element(s) selected from the group of boron, phosphorus, antimony and sulphur, more preferably, comprises, or consists of sulphur.

In case promoter P1 comprises, or consists of, sulphur, the precursor may be ammonium sulphate, but other sulphur precursors may also be used.

Preferably, the amount of the precursor of promoter P1 is selected such that the element(s) selected from boron, germanium, nitrogen, phosphorus, arsenic, antimony, sulphur, selenium and tellurium is(are) present in an amount of at least 0.02 g/100 g Fe, more preferably of at least 0.025 g/100 g Fe and most preferably of at least 0.05 g/100 g Fe in the catalyst.

Preferably, the amount of the precursor of promoter P1 is selected such that the element(s) selected from boron, germanium, nitrogen, phosphorus, arsenic, antimony, sulphur, selenium and tellurium is(are) present in an amount of not more than 0.25 g/100 g Fe, more preferably of not more than 0.20 g/100 g Fe and most preferably of not more than 0.15 g/100 g Fe in the catalyst.

The precursor of the promoter P2 is preferably selected from sodium hydroxide, sodium carbonate, sodium oxide, potassium hydroxide, potassium carbonate, potassium oxide, caesium hydroxide, caesium carbonate, caesium oxide or mixtures thereof, more preferably, is selected from sodium hydroxide, sodium carbonate, sodium oxide or mixtures thereof and most preferably is sodium carbonate.

The alkali metal- and/or alkaline-earth metal-containing promoter may contain more than one type of alkali metal- and/or alkaline-earth metal. The alkali metal- and/or alkaline-earth metal-containing promoter of the catalyst usually is in the form of an alkali metal oxide and/or alkaline-earth metal oxide.

Preferably, the alkali metal in the alkali metal-containing promoter is Na or K, and hence the alkali metal-containing promoter preferably comprises potassium oxide or sodium oxide.

Preferably, the alkaline-earth metal in the alkaline-earth metal-containing promoter is Ca or Mg.

Preferably, the amount of the precursor of promoter P2 is selected such that the alkali metal and/or alkaline-earth metal is/are present in an amount of at least 0.02 g/100 g Fe, more preferably of at least 0.06 g/100 g Fe and most preferably of at least 0.10 g/100 g Fe in the catalyst.

Preferably, the amount of the precursor of promoter P2 is selected such that the alkali metal and/or alkaline-earth metal is/are present in an amount of not more than 1.0 g/100 g Fe, more preferably of not more than 0.80 g/100 g Fe and most preferably of not more than 0.60 g/100 g Fe in the catalyst.

In an embodiment of the invention the alkali metal- or alkaline-earth metal-containing promoter and the at least one further promoter are chemically bound to another, in the form of a single compound. Preferably in this embodiment, the single compound is NaS.

Preferably, the weight ratio between the total of alkali metal and/or alkaline-earth metal of promoter P2 and the element(s) selected from boron, germanium, nitrogen, phosphorus, arsenic, antimony, sulphur, selenium and tellurium of promoter P1 in the catalyst is from 0.1:1 to 50:1, more preferably is from 0.5:1 to 33:1 and most preferably is from 0.8:1 to 20:1.

The catalyst obtained through the process of the invention after step (c) or step (d) is still in unreduced form and has to be reduced before being usable for hydrocarbon synthesis.

The reduction of the catalyst may be carried out in-situ. In this context "in-situ" denotes that the catalyst is placed in the reaction zone of the hydrocarbon synthesis process, e.g. a fluidized bed reactor or a fixed fluidized bed reactor, and reduced before the hydrocarbon synthesis reaction conditions are applied to said reaction zone.

Preferably, the reduction is carried out using H₂ and/or CO as reducing gas, more preferably H₂ is used as reducing gas.

Preferably the reduction is carried out at a temperature of at least 200 °C, more preferably of at least 275 °C, still more preferably of at least 300 °C, still more preferably of at least 350 °C, and most preferably of at least 380 °C.

The reduction is preferably carried out at a temperature of not more than 800 °C, more preferably of not more than 650 °C and most preferably of not more than 500 °C.

Preferably, the reduction is carried out for at least 8 hours, more preferably for at least 11 hours and most preferably for at least 14 hours.

The reduction is preferably carried out for at most 24 hours, more preferably for at most 21 hours and most preferably for at most 18 hours.

Preferably, the reduction is carried out at a pressure of at least 1.0 MPa, more preferably of at least 1.3 MPa and most preferably of at least 1.6 MPa.

The reduction is preferably carried out at a pressure of not more than 3.0 MPa, more preferably of not more than 2.7 MPa and most preferably of not more than 2.4 MPa.

During the reduction step, at least some of the Fe is reduced from e.g. an Fe-oxide to Fe in the zero oxidation state. Preferably, at least 75 wt.% of the Fe is reduced to Fe in the zero oxidation state.

It will be appreciated that the concentrations of the alkali metal and/or alkaline earth metal in the alkali metal- and/or alkaline-earth metal-containing promoter and the element(s) in the further promoter comprising one or more element(s) selected from the group of boron, silicon, germanium, nitrogen, phosphorus, arsenic, antimony, sulphur, selenium and tellurium are not changed during the reduction step, as these are expressed as g/100g Fe.

The catalyst in its reduced form preferably has a surface area of not more than 30 m²/g, more preferably of not more than 20 m²/g, and most preferably of not more than 10 m²/g. Usually, the surface area of the catalyst in its reduced form is not less than 1 m²/g.

The amount of Fe in the catalyst in its reduced form preferably is from 50 to 99.0 wt%, more preferably from 60 to 90 wt.%. The balance in the reduced form of the catalyst, excluding Fe and promoters, may be made up of carbon in the form of carbide.

Preferably, the catalyst is an unsupported catalyst, thus, preferably no catalyst support is added during the preparation process.

Preferably, in the catalyst in its reduced form promoter P2 is in the form of an alkali metal oxide and/or an alkaline-earth metal oxide.

The invention is furthermore directed to a hydrocarbon synthesis catalyst obtainable according to any of the above described embodiments of the process for the preparation of the catalyst.

Still further, the present invention is directed to a hydrocarbon synthesis process, especially a Fischer-Tropsch process, in the presence of the catalyst obtainable by the process in any of the above described embodiments, and to the use of the catalyst in such a hydrocarbon synthesis, especially Fischer-Tropsch, process.

Preferably, the hydrocarbon synthesis process is a high temperature process, more preferably is a high temperature Fischer-Tropsch process.

The high temperature hydrocarbon synthesis process is usually carried out at a temperature of 250 °C or higher, more preferably of 280 °C or higher, and most preferably of 300 °C or higher.

Preferably, the process is carried out at a temperature of 450 °C or lower, more preferably of 400 °C or lower, and most preferably of 360 °C or lower.

The total pressure in the reaction zone preferably is 5 bar or higher, more preferably is 10 bar or higher, and most preferably is 15 bar or higher.

Preferably, the total pressure is 60 bar or lower, more preferably is 50 bar or lower, still more preferably is 40 bar or lower, and most preferably is 30 bar or lower.

The high temperature hydrocarbon synthesis process can be described as a two-phase process. At the temperatures and pressures used for this process, both reactants and the products are in the gas phase in the reaction zone, and the catalyst, which is a solid, forms the second phase.

The applicant has further observed that under the preferred high temperature Fischer Tropsch reaction conditions described hereinbefore, the catalyst produces remarkably low levels of free carbon. High levels of free carbon formation is a known disadvantage associated with commercial high temperature Fischer Tropsch processes, which leads to carbon deposition on the catalyst surface and therefore deactivation of the catalyst. As a result, commercial processes employ fluidized bed reactors to avoid blocking of the catalyst bed.

In a preferred embodiment of this invention, the hydrocarbon synthesis process, preferably the Fischer Tropsch process is conducted in a stationary bed reactor.

The stationary bed reactor may define a zone wherein the catalyst is held stationary within the reactor. The zone may be defined by a first and second screen, carrying the catalyst, wherein the apertures of the screens are too small for the catalyst particles to pass through.

The stationary reactor may be a fixed bed reactor or a fixed-fluidised bed reactor, but preferably, the stationary bed reactor is a fixed bed reactor.

Preferably, the feed gas in the hydrocarbon synthesis process comprises at least one carbon oxide, more preferably comprises CO. The carbon oxide may comprise a mixture of CO and CO₂.

Furthermore, the feed gas preferably comprises H₂.

It is typical for a Fischer-Tropsch process that the feed gas both contains H₂ and carbon monoxide.

Preferably, the H₂:carbon oxide molar ratio is higher than 2:1, e.g. between 2.5:1 and 3:1, or may even be higher than 3:1.

The H₂ and carbon oxide feed is known as synthesis feed gas and it may also include other components, such as water vapour, Ar, CH₄, light hydrocarbons, etc.

The invention will now be described by means of the following non-limiting examples.

### Examples

### 1. Methods

### 1.1 Surface Area

Surface Area measurements were done using a Micromeritics Tristar 3000. Samples were degassed under dynamic nitrogen flow at 200 °C for a minimum time of 12 hours. Thereafter 250 - 300 mg of sample were accurately weighed out and loaded into 3/8 inch tubes onto the instrument. Sample tubes were immersed in a liquid nitrogen bath and evacuated. Leak tests were conducted, where after a total of eight relative pressure points were measured in a range from 0.08 to 0.98. Measured parameters were used by the instrument software to calculate the surface area, total pore volume and average pore diameter of the samples.

### 1.2 Particle Size

The Particle size of the catalyst particles is determined using fine test sieves fitted with stainless steel wire cloth meeting ASTM specification E-11.

### 1.3 Catalyst Preparation Procedure A (inventive Example 1)

For the reverse precipitation of iron, up to 100 ml 25% (v/v) NH₄OH solution was added drop-wise, whilst stirring with an overhead stirrer, to 400 ml of 1 M aqueous solution of Fe(NO₃)₃·9H₂O (161.6 g) containing the appropriate amount of the selected non-metal precursor of promoter P1 until a pH of 7 at room temperature (25 °C) was reached. Thereafter, Na₂CO₃ was added in the appropriate amounts to the precipitation mixture. The resultant slurry was then dried in a fan-oven overnight (approximately 16 hours) at 150 °C, and then calcined in air at 350 °C for 4 hours. Finally, the catalyst was crushed and screened to a particle size range of 38-150 µm. This was achieved by screening the catalyst over a 38 µm sieve and discarding the <38 µm fraction, followed by screening over a 150 µm sieve and discarding the >150 µm fraction using fine test sieves fitted with stainless steel wire cloth meeting ASTM specification E-11.

### 1.4 Catalyst Preparation Procedure B (comparative Example 2)

For the reverse precipitation of iron, up to 100 ml 25% (v/v) NH₄OH solution was added drop-wise, whilst stirring with an overhead stirrer, to 400 ml of 1 M aqueous solution of Fe(NO₃)₃·9H₂O (161.6 g) until a pH of 7 at room temperature (25 °C) was reached. Thereafter, Na₂CO₃ was added in the appropriate amounts to the precipitation mixture. The resultant slurry was then dried in a fan-oven overnight (approximately 16 hours) at 150 °C, and then calcined in air at 350 °C for 4 hours. The catalyst was then crushed and screened to a particle size range of 38-150 µm using fine test sieves fitted with stainless steel wire cloth meeting ASTM specification E-11. Thereafter the iron oxide particles were suspended in a water mixture containing the appropriate amount of the selected non-metal precursor of promoter P1 and agitated for approximately 30 minutes. The resultant slurry was then dried in a fan-oven overnight (approximately 16 hours) at 150 °C, and again calcined in air at 350 °C for 4 hours.

### 1.5 Catalyst Preparation Procedure C (comparative Example 3)

For the reverse precipitation of iron, up to 100 ml 25% (v/v) NH₄OH solution was added drop-wise, whilst stirring with an overhead stirrer, to 400 ml of 1 M aqueous solution of Fe(NO₃)₃·9H₂O (161.6 g) until a pH of 7 at room temperature (25 °C) was reached. Thereafter, Na₂CO₃ was added in the appropriate amounts to the precipitation mixture. The resultant slurry was then dried in a fan-oven overnight (approximately 16 hours) at 150 °C, and then calcined in air at 350 °C for 4 hours. Finally, the catalyst was crushed and screened to a particle size range of 38-150 µm using fine test sieves fitted with stainless steel wire cloth meeting ASTM specification E-11.

### 1.6 Catalyst Testing Procedure

5 g calcined catalyst was loaded into a berty type gradientless micro fixed bed reactor and reduced *in situ* under hydrogen at 420 °C for 16 hours at 20 bar. Thereafter, synthesis gas was introduced at a flow rate of 13 liters (n) per g catalyst per hour (H₂ = 57 volume %, CO = 14 volume %, CO₂ = 11 volume %) at 20 bar total pressure and at a temperature of 330 °C. Analysis of hydrocarbon products was performed using GC-FID, and permanent gas analysis was done by GC-TCD.

### 1.7 Selectivity

All selectivities are expressed as carbon-atom % selectivity and are not normalized. CO₂ formation is excluded in selectivity calculations.

### 2. Experiments

### Inventive Example 1

Following the general catalyst preparation procedure A described above, a catalyst with a composition of 0.419 g Na per 100 g Fe and 0.112 g S per 100 g Fe was prepared and tested in a Berty type gradientless micro reactor as described in the general catalyst testing methodology above. The sulphur precursor used was ammonium sulphate. The synthesis results are summarized in Table 1.

### Comparative Example 2

Following the general catalyst preparation procedure B described above a catalyst with a composition of 0.419 g Na per 100 g Fe and 0.112 g S per 100 g Fe was prepared and tested in a Berty type gradientless micro reactor as described in the general catalyst testing methodology above.

The sulphur precursor used was ammonium sulphate. The synthesis results are summarized in Table 1.

### Comparative Example 3

Following the general catalyst preparation procedure C described above a catalyst with a composition of 0.295 g Na per 100 g Fe was prepared and tested in a Berty type gradientless microreactor as described in the general catalyst testing procedure above. The synthesis results are summarized in Table 1.

**Table 1:**

| | Example 1† | Comparative Example 2* | Comparative Example 3† |
|---|---|---|---|
| Na | 0.419 g | 0.419 g | 0.295 g |
| S | 0.112 g | 0.112 g | - |
| Fe | 100 g | 100 g | 100 g |
| CO+H₂ conversion | 33.5 % | 39.8 % | 46 % |
| CO+CO₂ conversion | 33.9 % | 38.3 % | 44 % |
| CH₄ selectivity | 10.8 % | 14.1 % | 10 % |
| C2-C4 selectivity | 59.3 % | 58.2 % | 34 % |
| C2-C8 selectivity | 76.4 % | 78.6 % | 60 % |
| C5+ selectivity | 22.3 % | 24.5 % | 46 % |
| olefins in C2-C4 fraction | 61.6 % | 63.7 % | 70 % |
| alcohols in C2-C4 fraction | 22.1 % | 15.8 % | 13 % |
| paraffins in C2-C4 fraction | 9.7 % | 14.3 % | 17 % |
| Surface area (unreduced catalyst) | 27 m²/g | 15 m²/g | 32.8 m²/g |

| | | | |
|---|---|---|---|
| t Estimated mass balance: 95%; * Estimated mass balance: 97 % | | | |

### Discussion of the results in Table 1:

Surprisingly, when comparing the synthesis performance of the catalyst of Comparative Example 3 (sulphur free) with the catalysts promoted by sulphur in combination with alkali metal (Example 1), it will be noted that the methane selectivity remained constant while a dramatic increase in selectivity for light hydrocarbons (C₂-C₄ hydrocarbon fraction) was observed.

Comparative Example 2 demonstrates that it surprisingly makes a difference whether the calcined catalyst is impregnated with sulphur as in Comparative Example 2 or whether a sulphur precursor is embedded in the catalyst structure as in Example 1 because the undesired methane formation of the impregnated catalyst is higher.

## Claims

1. Process for the preparation of a hydrocarbon synthesis catalyst comprising the following steps
(a) providing a solution of iron or a suspension of a precipitated, non-calcined iron-containing solid in a solvent in which a precursor of a promoter P1 is present, the promoter P1 comprising one or more element(s) selected from the group of boron, germanium, nitrogen, phosphorus, arsenic, antimony, sulphur, selenium and tellurium,
(b) removing the solvent from the solution or suspension, and
(c) subjecting the product of step (b) to a calcination treatment,
wherein
- the amount of the precursor of promoter P1 is selected so that the one or more element(s) selected from boron, germanium, nitrogen, phosphorus, arsenic, antimony, sulphur, selenium and tellurium is(are) present in an amount of at least 0.02 g/100 g Fe in the catalyst, and
- a precursor of an alkali metal- and/or alkaline earth metal containing promoter P2 is added before, after or during any of the steps of the process so that promoter P2 is present in the catalyst.

2. Process according to claim 1, wherein in step (a) a solution of iron in a solvent is provided in which a precursor of a promoter P1 is present, and then a suspension is obtained by forming a precipitate containing iron and the precursor of promoter P1 from the solution before step (b) is carried out.

3. Process according to claims 1 or 2 wherein the solvent is an aqueous solvent.

4. Process according to any one of the preceding claims wherein the calcination treatment in step (c) is performed at a temperature of 200 °C or more.

5. Process according to any one of the preceding claims, wherein the product of step (b) has surface area of 50 to 500 m²/g.

6. Process according to any one of the preceding claims, wherein the product of step (c) has surface area of 10 to 80 m²/g.

7. Process according to any one of the preceding claims wherein the process further comprises the following step
(d) the product of step (c) is brought into a particulate form in which more than 90 wt.% of the particles have a particle size in the range of 10 to 250 micrometer.

8. Process according to any one of the preceding claims, wherein promoter P1 comprises one or more element(s) selected from the group of boron, phosphorus, antimony and sulphur.

9. Process according to claim 8, wherein promoter P1 comprises sulphur.

10. Process according to any one of the preceding claims, wherein the amount of the precursor of promoter P1 is selected so that the one or more element(s) selected from boron, germanium, nitrogen, phosphorus, arsenic, antimony, sulphur, selenium and tellurium is(are) present in an amount of not more than 0.25 g/100 g Fe in the catalyst

11. Process according to any one of the preceding claims, wherein the amount of the precursor of promoter P2 is selected such that the alkali metal and/or alkaline-earth metal is/are present in an amount of from 0.02 to 1.0 g/100 g Fe in the catalyst.

12. Process according to any one of the preceding claims, wherein the precursor of promoter P2 is selected from sodium hydroxide, sodium carbonate, sodium oxide, potassium hydroxide, potassium carbonate, potassium oxide, caesium hydroxide, caesium carbonate, caesium oxide or mixtures thereof.

13. Catalyst obtainable by the process according to any of the preceding claims.

14. Hydrocarbon synthesis process which is carried out in the presence of a catalyst according to claim 13.

15. Use of a catalyst according to claim 13 in a hydrocarbon synthesis process.
